# EUROPÄISCHE PATENTANMELDUNG

(11) **EP 0 673 660 A2**
(43) Veröffentlichungstag der Anmeldung: **27.09.1995**
(21) Anmeldenummer: 94114280.4
(22) Anmeldetag: 12.09.1994
(51) Int. Cl.: A61N 1/05, A61N 1/04

(54) **Elektroden-Vorrichtung für die elektronische Anästhesie im Mundbereich**

(30) Priorität: 25.03.1994 DE 9405127 U
(71) Anmelder: Schramm, Heinrich W., D-33803 Steinhagen (DE)
(72) Erfinder: Schramm, Heinrich W., D-33803 Steinhagen (DE)
(74) Vertreter: TER MEER - MÜLLER - STEINMEISTER & PARTNER

(57) **Zusammenfassung**

Eine Elektroden-Vorrichtung für die elektronische Anästhesie im Mundbereich umfaßt einen elastisch verformbaren, U-förmigen oder ringförmigen Trägerbügel (10,22), eine auf dem Trägerbügel angebrachte Elektrode (16,32) und eine von der Elektrode ausgehende, zu einem Steuergerät führende Verbindungsleitung (18,36).

## Beschreibung

Die Erfindung betrifft eine Elektroden-Vorrichtung für die elektronische Anästhesie im Mundbereich.

Bei der zahnärztlichen Behandlung ist es üblich, vor der Durchführung von schmerzerzeugenden Behandlungen ein Anästhetikum zu injizieren. Viele Menschen reagieren jedoch allergisch auf Anästhetika oder empfinden die Taubheit nach der Behandlung als unangenehm. Auch die Injektion selbst wird als schmerzhaft empfunden. Die daraus resultierende Abwehrhaltung wirkt auf den Zahnarzt psychisch belastend. Schließlich stellen die bei der Injektion verwendeten Spritzen, Nadeln und Ampullen zumeist Einwegartikel dar, deren Beseitigung nach der Verwendung Probleme bereitet.

In zunehmendem Maße wird daher die sogenannte elektronische Anästhesie eingesetzt, bei der durch geringe Ströme, die über eine Elektrode im Mundbereich und eine weitere Elektrode in einen anderen Bereich des Körpers des Patienten eingeleitet werden. Obgleich die Wirkung der elektronischen Anästhesie wissenschaftlich nicht vollständig geklärt ist, wird angenommen, daß sie darauf berunht, daß die schmerzleitenden Nerven blockiert und körpereigene Opiate (Endorphine und Enzephaline) gebildet werden. Die zuverlässige Anbringung von Elektroden im Mundbereich hat jedoch bisher Schwierigkeiten bereitet.

Da sich die von einem Zahn ausgehenden Nerven in den zugehörigen Kiefer erstrecken, ist es erforderlich, eine Elektrode an der Außenseite des Kiefers in der Position des zu behandelnden Zahnes anzubringen. Haftkleber-Elektroden, die in anderen Bereichen des Körpers mit Erfolg eingesetzt werden können, bieten wegen der im Mundbereich bestehenden Feuchtigkeit zumindest nur für eine sehr begrenzte Zeit den notwendigen Halt. Es ist daher auch üblich, Haftkleber-Elektroden an der Außenseite des Gesichts außerhalb der betroffenen Kieferposition anzubringen. Hier ist jedoch die Stromübertragung zum betroffenen Bereich des Kiefers oft unzureichend.

Der Erfindung liegt daher die Aufgabe zugrunde, eine Elektroden-Vorrichtung zu schaffen, die eine sichere und unmittelbare Übertragung der anzulegenden Ströme in der gewünschten Position des betroffenen Kieferbereichs gestattet.

Diese Aufgabe wird erfindungsgemäß gelöst durch einen elastisch verformbaren U-förmigen oder ringförmigen Trägerbügel, eine auf dem Trägerbügel angebrachte Elektrode und eine von der Elektrode ausgehende, zu einem Steuergerät führende Verbindungsleitung.

Sofern der Trägerbügel U-förmig ausgebildet ist, kann er in die obere bzw. untere Kieferntasche um den jeweiligen Kiefer herum eingelegt werden. Aus einem ringförmigen Trägerbügel werden durch Zusammenklappen gewissermaßen zwei U-förmige Bügel gebildet, die in beide Kieferntaschen von Ober- und Unterkiefer eingelegt werden und sich dabei gegenseitig abstützen, so daß ein Herausrutschen aus der betroffenen Kieferntasche verhindert wird. Zur Anpassung an die Größe des Patienten kann ein ringförmiger Trägerbügel verstellbar sein, indem er beispielsweise einander überlappende, offene Enden aufweist, die innerhalb einer geeigneten Hülle gegeneinander verschoben werden können.

Als Material für den Trägerbügel kommen nichtrostender, federnder Stahldraht oder auch Kunststoff in Betracht. Bei Verwendung eines leitenden Materials, wie etwa Stahldraht, befindet sich auf zumindest einem Abschnitt des Trägerbügels ein isolierender Schlauch, beispielsweise Silikonschlauch, der seinerseits die Elektrode trägt. Der Schlauch ist zusammen mit der Elektrode auf dem Trägerbügel in die gewünschte Position verschiebbar.

Im folgenden werden bevorzugte Ausführungsbeispiele der Erfindung anhand der beigefügten Zeichnung näher erläutert.
- Fig. 1: ist eine schematische perspektivische Darstellung einer ersten Ausführungsform der Erfindung;
- Fig. 2: ist eine entsprechende Darstellung einer zweiten Ausführungsform der Erfindung;
- Fig. 3: veranschaulicht schematisch die Gestaltung eines ringförmigen, in der Größe verstellbaren Trägerbügels.

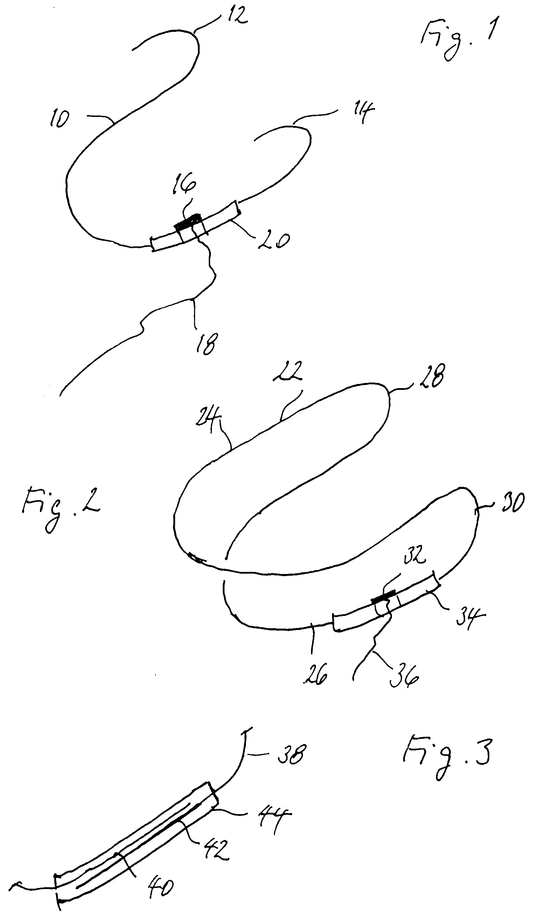

Fig. 1 zeigt einen U-förmigen Trägerbügel 10, der beispielsweise aus Stahldraht besteht. Die beiden offenen Enden 12,14 des Trägerbügels sind ihrerseits U-förmig umgebogen. In der dargestellten Orientierung kann der Trägerbügel 10 beispielsweise in die Kieferntasche des Unterkiefers eines Patienten eingelegt werden. Dabei dienen die umgebogenen Enden 12,14 zur Abstützung in der Kieferntasche des Oberkiefers. Auf diese Weise wird verhindert, daß sich der Bügel 10 während der zahnärztlichen Arbeiten anhebt.

Auf den Trägerbügel 10 ist eine in Längsrichtung verschiebbare Elektrode 16 angebracht, die über eine elektrische Leitung 18 mit einem nicht gezeigten Steuergerät verbunden ist. Sofern der Trägerbügel 10 aus einem leitenden Material, etwa Stahldraht besteht, ist eine Isolierung zwischen der Elektrode 16 und dem Trägerbügel 10 erforderlich. Aus diesem Grunde sitzt die Elektrode 16 im dargestellten Beispiel auf einem isolierenden Schlauchabschnitt 20, beispielsweise aus Silikongummi.

Der in Fig. 2 gezeigte Trägerbügel 22 ist als geschlossener Ring ausgebildet. Dieser Ring, der beispielsweise wiederum aus elastischem, biegsamem Material wie Stahldraht besteht, kann zur Bildung von zwei U-förmigen Abschnitten 24 und 26 zusammengeklappt werden. Dabei dient der in der Zeichnung oben liegende Abschnitt 24 zum Einlegen in die Kieferntasche des Oberkiefers, während der untere Abschnitt 26 für den Unterkiefer vorgesehen ist. In dieser Position befinden sich die beiden bogenförmigen Übergänge 28 und 30 im hinteren Bereich des Mundes, in dem sie die zahnärztlichen Arbeiten nicht stören. Durch die federnden Eigenschaften des Materials des Trägerbügels und die Zusammenlegung in der in Fig. 2 gezeigten Form werden der obere und untere Abschnitt 24 und 26 auseinandergespannt, so daß sie fest in die jeweiligen Kieferntaschen hineingedrückt werden.

Auch bei der in Fig. 2 gezeigten Ausführungsform befindet sich wiederum eine Elektrode 32 auf einem Schlauchabschnitt 34, der auf dem Trägerbügel 22 verschiebbar ist. Eine Leitung 36 dient zur Verbindung mit dem Steuergerät.

Ein Trägerbügel 22 gemäß Fig. 2 kann in unterschiedlichen Größen bereitgehalten werden, oder es kann auch ein verstellbarer Trägerbügel 38 verwendet werden, der in Fig. 3 nur teilweise angedeutet ist. Dieser Trägerbügel weist zwei offene, einander überlappende Enden 40,42, die in einer Hülle 44 geführt sind, auf. Durch gegenseitiges Verschieben der Enden 40,42 in der Hülle 44 kann der Umfang oder Durchmesser des ringförmigen Trägerbügels 28 verstellt werden. Dabei können die Enden 40,42 innerhalb der Hülle 44 beispielsweise gebremst sein, so daß sie in der durch Verschiebung erreichten Stellung ohne zusätzliche Halte- oder Klemmeinrichtungen gehalten werden.

## Patentansprüche

1. Elektroden-Vorrichtung für die elektronische Anästhesie im Mundbereich, **gekennzeichnet** durch einen elastisch verformbaren, U-förmigen oder ringförmigen Trägerbügel (10,22), eine auf dem Trägerbügel angebrachte Elektrode (16,32) und eine von der Elektrode ausgehende, zu einem Steuergerät führende Verbindungsleitung (18,36).

2. Elektroden-Vorrichtung nach Anspruch 1, dadurch **gekennzeichnet**, daß die Elektrode (16,32) auf dem Trägerbügel (10,22) längsverschiebbar ist.

3. Elektroden-Vorrichtung nach Anspruch 1 oder 2, dadurch **gekennzeichnet**, daß der Trägerbügel (10,22) aus Stahldraht oder einem anderen leitenden Material besteht, daß auf dem Trägerbügel ein längsverschiebbarer, biegsamer Schlauchabschnitt (20,34) aus nichtleitendem Material angeordnet ist, und daß sich auf dem Schlauchabschnitt die Elektrode (16,32) befindet.

4. Elektroden-Vorrichtung nach einem der Ansprüche 1 bis 3, dadurch **gekennzeichnet**, daß der Trägerbügel (38) durch ringförmiges, überlappendes Zusammenlegen der beiden Enden (40,42) eines Drahtabschnitts gebildet ist, und daß die beiden Enden (40,42) gegeneinander verschiebbar in einer Hülle (44) gehalten sind.
